(19) Europäisches Patentamt
European Patent Office
Office européen des brevets

(11) Publication number: 0 081 094
A1

(12) # EUROPEAN PATENT APPLICATION

(21) Application number: 82110398.3

(22) Date of filing: 11.11.82

(51) Int. Cl.[3]: C 07 C 103/52
A 61 K 37/02

(30) Priority: 12.11.81 US 320263

(43) Date of publication of application: 15.06.83 Bulletin 83/24

(84) Designated Contracting States: CH DE FR GB IT LI NL

(71) Applicant: MERCK & CO. INC.
126, East Lincoln Avenue P.O. Box 2000
Rahway New Jersey 07065(US)

(72) Inventor: Patchett, Arthur A.
1090 Minisink Way
Westfield New Jersey 07090(US)

(72) Inventor: Wu, Mu Tsu
35 Lance Drive
Clark New Jersey 07066(US)

(74) Representative: Abitz, Walter, Dr.-Ing. et al,
Abitz, Morf, Gritschneder, Freiherr von Wittgenstein
Postfach 86 01 09
D-8000 München 86(DE)

(54) **Substituted omega-amino-carboxymethyldipeptide antihypertensive agents.**

(57) Compounds of the formulae:

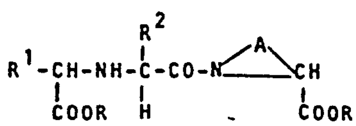

I

and

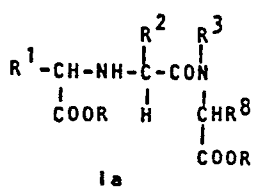

Ia

wherein:

R and $R^1$ are, e.g., hydrogen;

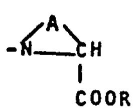

is, e.g.,

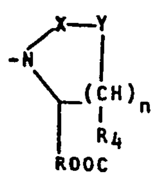

,

where

X and Y taken together are, e.g., $-CH_2-CH_2-$;
$R^4$ is hydrogen or loweralkyl; and
n is 1 to 3
$R^3$ is, e.g., $C_{3-8}$ cycloalkyl;
$R^8$ is hydrogen; loweralkyl;
$R^2$ is $-(CH_2)_r-B-(CH_2)_s-NHR^7$

where

r is 1-2;
s is 1-3;
B is, e.g., absent or $-O-$; and
$R^7$ is, e.g., loweralkyl;
and, a pharmaceutically acceptable salt thereof.
Those compounds are inhibitors of angiotensin converting enzyme and useful as antihypertensive agents.

EP 0 081 094 A1

TITLE OF THE INVENTION

SUBSTITUTED ω-AMINO-CARBOXYMETHYLDIPEPTIDE ANTI-HYPERTENSIVE AGENTS

BACKGROUND OF THE INVENTION

1. Field of the Invention

The present invention is concerned with substituted ω-amino carboxymethyldipeptide compounds which are effective inhibitors of angiotensin I converting enzyme. These novel compounds are, consequently, combined with pharmaceutically acceptable carriers to form pharmaceutical compositions of the present invention and are used in a method of treating hypertension.

Angiotensin II, a powerful vasoconstrictor hormonal peptide, is formed from the inactive angiotensin I by the action of angiotensin-converting enzyme. Recently, potent inhibitors of angiotensin-converting enzyme have been reported

which are capable of lowering the blood pressure in hypertensive patients. The compounds of the present invention are also potent inhibitors of angiotensin converting enzyme.

2. Brief Description of the Prior Art

U.S. Patents 4,113,715; 4,129.571; and 4,154,960 disclose substituted acyl derivatives of amino acids which are useful as angiotension converting enzyme inhibitors. More specifically, these compounds are mercapto substituted acyl amino acids an derivatives thereof including the clinically effective antihypertensive compound, captopril, i.e., D-3-mercapto-2-methylpropanoyl-L-proline.

The foregoing prior art compounds are not dipeptide derivatives as are the compounds of the present invention. Furthermore, these prior art compounds contain an essential sulfhydryl substituent or derivative thereof whereas those of the present invention do not. In addition, the dipeptide compounds of the present invention are unusual dipeptides whose N-terminus bears a carboxymethyl group which is preferably further substituted on the methyl group. In addition, the carboxyl group(s) may also be converted to ester, amide and salt derivatives. In effect, the compounds of the present invention are hybrids formed by fusing α-amino acids onto dipeptides by means of a nitrogen shared by these two part-structures. This structural arrangement is rare in the field of synthetic and natural peptides and is not suggested or disclosed by the mercaptoacyl type functions of the two prior art patents identified above.

U.S. Patent 4,052,511 discloses N-carboxyalkanoyl-amino acids which are useful as angiotension converting enzyme inhibitors. Since the compounds of the present invention are dipeptide derivatives, in a formal sense they may be considered to be related to some of the compounds disclosed in U.S. Patent 4,052,511. However, when a particular one of the methylene groups is replaced by an amino function as in the present invention, compounds of surprisingly high potency are obtained.

## DETAILED DESCRIPTION OF THE INVENTION AND PREFERRED EMBODIMENTS

In its broadest aspect, the present invention relates to substituted ω-amino-carboxymethyldipeptide compounds of the formulae:

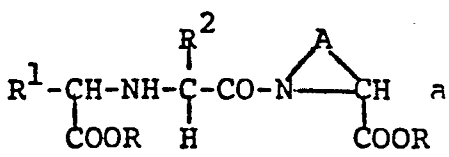
and
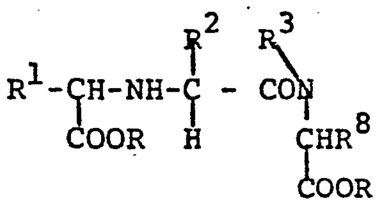

(I.) (Ia)

wherein:

R is hydrogen; loweralkyl; aralkyl; aryl;

$R^1$ is hydrogen; $C_{1-12}$ alkyl; $C_{3-9}$ cycloalkyl; $C_{1-12}$ alkenyl; substituted loweralkyl where the substituents are halo, hydroxy loweralkoxy, aryloxy, amino, mono- or diloweralkylamino, acylamino, arylamino,

guanidino, mercapto, loweralkylthio, arylthio, carboxy, carboxamido, or loweralkoxycarbonyl; aryl; substituted aryl where the substituents are loweralkyl, loweralkoxy, or halo; arloweralkyl; arloweralkenyl; heteroarloweralkyl; heteroarloweralkenyl; substituted arloweralkyl, substituted arloweralkenyl, substituted heteroarloweralkyl, or substituted heteroarloweralkenyl where the aryl and heteroaryl substituents are halo, loweralkyl, hydroxy, loweralkoxy, amino, aminoloweralkyl, acylamino, mono- or diloweralkylamino, carboxyl, haloloweralkyl, cyano, or sulfonamido, and where the loweralkyl portion of arloweralkyl may be substituted by amino, acylamino, or hydroxyl;

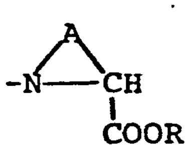 is 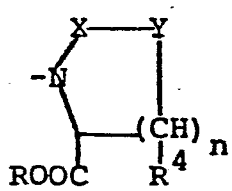 , 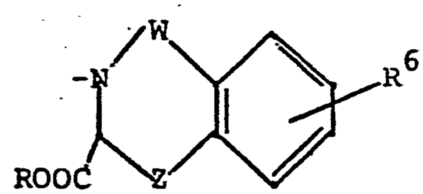

or 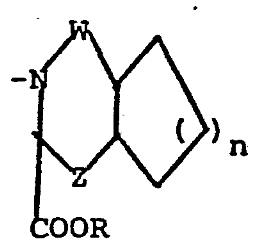

where

X and Y taken together are $-CH_2-CH_2-$;

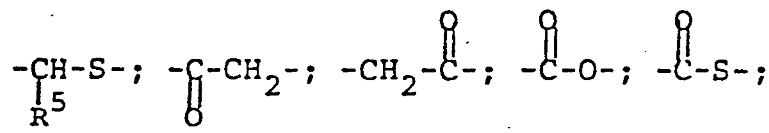

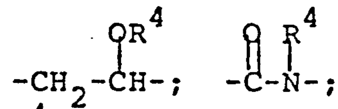 or $-CH_2CR^4R^5-$

| | |
|---|---|
| $R^4$ is | hydrogen or loweralkyl; |
| $R^5$ is | hydrogen; loweralkyl; aryl; or substituted aryl; |
| n is | 1 to 3; |
| W is | absent; $-CH_2-$; or 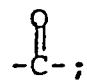 |
| Z is | $-(CH_2)_m-$, where m is 0 to 2, provided that m may not be 0 and W may not be absent at the same time; and |
| $R^6$ is | hydrogen; loweralkyl; halo; or $OR^4$; |
| $R^3$ is | $C_{3-8}$ cycloalkyl and benzofused derivatives thereof; aryl; heteroaryl: aralkyl; heteroaralkyl; |
| $R^8$ is | hydrogen; loweralkyl; |

$R^2$ is $-(CH_2)_r-B-(CH_2)_s-NHR^7$

where

r is 1-2;

s is 1-3;

B is absent; -O-; -S-; or $-NR^9-$;

where $R^9$ is hydrogen; loweralkyl; alkanoyl; or aroyl; and

$R^7$ is loweralkyl; loweralkenyl; loweralkynyl; substituted loweralkyl wherein

the substituent is amino, alkyl-and dialkylamino, halo, alkoxy or arylthio; aralkyl; substituted aralkyl wherein the substituent is halo, alkoxy, or hydroxy; heteroaralkyl; substituted heteroaralkyl wherein the substituent is amino, hydroxy, or loweralkyl;

and, a pharmaceutically acceptable salt thereof.

The loweralkyl, loweralkenyl, and loweralkynyl substituents recited above represent, except where otherwise indicated, straight and branched chain hydrocarbon radicals of from one to six carbon atoms, such as methyl, ethyl, n-propyl, i-propyl, n-butyl, i-butyl, t-butyl, n-hexyl; or vinyl, allyl, butenyl, propargyl, 2-butinyl and the like.

The loweralkoxy substituent represents a loweralkyl group as described above attached through an oxygen bridge.

The aryl substituent represents phenyl naphthyl, or biphenyl. Substituted aryl are substituted by halo, alkoxy, or methyl.

The arloweralkyl and arloweralkenyl substituents recited above represent phenyl or naphthyl attached through a straight or branched chain hydrocarbon of from one to six carbon atoms, for example, benzyl.

Halo means chloro, bromo, iodo, or fluoro.

The heteroaryl substituent recited above represents any 5- or 6-membered aromatic ring containing from one to three heteroatoms selected

from the group consisting of nitrogen, oxygen, and sulfur, for example, pyridyl, thienyl, furyl, imidazolyl, and thiazolyl; as well as any bicyclic group in which any of the above heterocyclic rings is fused to a benzene ring, for example, indolyl, quinolinyl, isoquinolinyl, benzimidazolyl, benzothiazolyl, and benzthienyl.

The acylamino substituent represents loweralkanoylamino and aroylamino.

Where 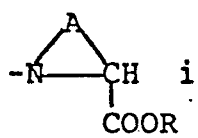 is 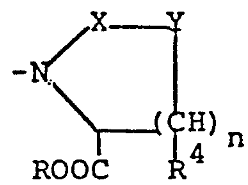 ,

the heteroaryl element: 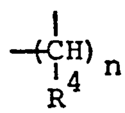

provides for expansion of the ring where n is greater than 1 and $R^4$ is hydrogen, or for loweralkyl substitution of the ring where $R^4$ is loweralkyl, or for any combination of both.

The Formulae I and Ia compounds can be used in the form of salts derived from inorganic or organic acids. Included among such salts are the following: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfoate, fumarate, glucoheptanoate, glycerophosphate, hemisulfate, heptanoate, hexanoate, hydrochloride, hydrobromide, hydroiodide, 2-hydroxyethanesulfonate, lactate, maleate,

methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Also, when R=H, Formulae I and Ia compounds can be used as salts derived from inorganic or organic bases. These salts include sodium, potassium, calcium and ammonium.

Preferred compounds of the present invention are those of Formula I wherein:

R is hydrogen or loweralkyl;

$R^1$ is $C_{1-8}$ alkyl; substituted $C_{1-5}$ alkyl where the substituents are amino, arylthio, aryloxy, or arylamino; aralkyl or heteroaralkyl where the alkyl portion has 1 to 3 carbon atoms, for example phenethyl or imidazolylethyl; or substituted aralkyl or heteroaralkyl where the alkyl portion has 1 to 3 carbon atoms and the substituents are halo, loweralkyl, hydroxy, loweralkoxy, amino, or aminoloweralkyl, and where the alkyl portion of aralkyl may be substituted by amino, acylamino, or hydroxyl;

for $R^2$, B is absent, r and s are each 2, and $R^7$ is loweralkyl; arloweralkyl; heteroarloweralkyl; aminoloweralkyl; dialkylamino; loweralkyl; haloalkyl; or trihaloalkyl;

$R^3$ is $C_{5-8}$ cycloalkyl and benzofused derivatives thereof;

$R^8$ is hydrogen;

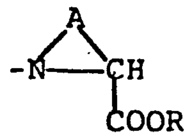 is 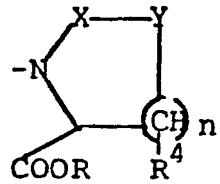

where X and Y taken together are -CH -CH -; $R^4$ is hydrogen; n is 1; and R is as previously defined;

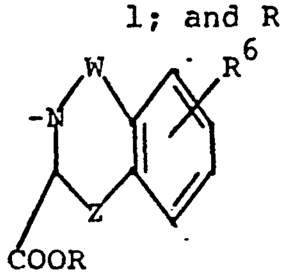

where

W is absent or $-CH_2-$;

Z is absent· $-CH_2-$, or $-CH_2CH_2$, provided W and Z are not both absent at the same time;

$R^6$ is hydrogen; and R is as defined above;

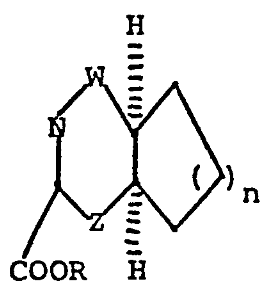

where

W is absent; $-CH_2-$;

Z is $-CH_2-$;

n is 2.

Especially preferred compounds of the present invention are the following:

$N^2$-[1-(S)-carboxy-3-phenylpropyl]-$N^6$-(methyl)-L-lysyl-L-proline;

$N^2$-[1-(S)-carboxy-3-phenylpropyl]-$N^6$-(2'-aminoethyl)-L-lysyl-L-proline;

$N^2$-[1-(S)-carboxy-3-phenylpropyl]-$N^6$-(2',2',2'-trifluoroethyl)-L-lysyl-L-proline;

$N^2$-[1-(S)-carboxy-3-phenylpropyl]-$N^6$-(2'-methoxyethyl)-L-lysyl-L-proline;

$N^2$-[1-(S)-carboxy-3-phenylpropyl]-$N^6$-((2'-dimethylaminoethyl)-L-lysyl-L-proline.

The compounds of this invention inhibit angiotensin converting enzyme and thus block conversion of the decapeptide angiotensin I to angiotensin II. Angiotensin II is a potent pressor substance. Thus blood-pressure lowering can result from inhibition of its biosynthesis especially in animals and humans whose hypertension is angiotensin II related. Furthermore, converting enzyme degrades the vasodilator peptide, bradykinin. Therefore, inhibitors of angiotensin converting enzyme may lower blood-pressure also by potentiation of bradykinin. Although the relative importance of these and other possible mechanisms remains to be established, inhibitors of angiotensin converting enzyme are effective antihypertensive agents in a variety of

animal models and are useful clinically, for example, in many human patients with renovascular, malignant and essential hypertension. See, for example, D. W. Cushman et al., Biochemistry 16, 5484 (1977).

The evaluation of converting enzyme inhibitors is guided by *in vitro* enzme inhibition assays. For example, a useful method is that of Y. Piquilloud, A. Reinharz and M. Roth, Biochem. Biophys. Acta, 206, 136 (1970) in which the hydrolysis of carbobenzyloxyphenylalanylhistidinylleucine is measured. *In vivo* evaluations may be made, for example, in normotensive rats challenged with angiotensin I by the technique of J. R. Weeks and J. A. Jones, Proc. Soc. Exp. Biol. Med., 104, 646 (1960) or in a high renin rat model such as that of S. Koletsky et al., Proc. Soc. Exp. Biol. Med., 125, 96 (1967).

Thus, the compounds of the invention are useful in treating hypertension. They are also of value in the management of acute and chronic congestive heart failure, in the treatment of secondary hyperaldosteronism, primary and secondary pulmonary hypertension, renal failure and renal vascular hypertension, and in the management of vascular disorders such as migraine. The application of the compounds of this invention for these and similar disorders will be apparent to those skilled in the art.

Thus, in accordance with the present invention there is provided a pharmaceutical composition comprising a pharmaceutically acceptable

carrier and a therapeutically effective amount of a compound of Formulae I or Ia.

There is also provided, in accordance with the present invention, a method of treating hypertension comprising administering to a patient in need of such treatment a therapeutically effective amount of a compound of Formulae I or Ia.

For the purpose of treating hypertension, and those clinical conditions noted above, the compounds of the present invention may be administered orally, topically, parenterally, by inhalation spray or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as mice, rats, horses, dogs, cats, etc., the compounds of the invention are effective in the treatment of humans.

The pharmaceutical compositions containing the active ingredient may be in a form suitable for oral use, for example, as tablets, troches, lozenges, aqueous or oily suspensions, dispersible powders or granules, emulsions, hard or soft capsules, syrups or elixirs. Compositions intended for oral use may be prepared according to any method known to the art for the manufacture of pharmaceutical compositions and such compositions may contain one or more agents selected from the group consisting of sweetening

agents, flavoring agents, coloring agents and preserving agents in order to provide a pharmaceutically elegant and palatable preparation. Tablets contain the active ingredient in admixture with non-toxic pharmaceutically acceptable excipients which are suitable for manufacture of tablets. These excipients may be, for example, inert diluents, such as calcium carbonate, sodium carbonate, lactose, calcium phosphate or sodium phosphate; granulating and disintegrating agents, for example maize starch, or alginic acid; binding agents, for example, starch, gelatine or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate alone or with a wax may be employed.

Formulations for oral use may also be presented as hard gelatine capsules wherein the active ingredient is mixed with an inert solid diluent, for example calcium carbonate, calcium phosphate or kaolin, or as soft gelatine capsules wherein the active ingredient is mixed with water or an oil medium, for example arachis oil, peanut oil, liquid paraffin or olive oil.

Aqueous suspensions contain the active materials in admixture with excipients suitable for the manufacture of aqueous suspensions. Such

excipients are suspending agents, for example sodium carboxymethyl cellulose, methylcellulose, hydroxypropylmethylcellulose, sodium alginate, polyvinylpyrrolidone, gum tragacanth and gum acacia; dispersing or wetting agents may be a natural-occurring phosphatide, for example, lecithin, or condensation products of an alkylene oxide with fatty acids, for example polyoxvethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol mono-oleate, or condensation products of ethylene oxide with partial esters derived from fatty acids and hexitol anhydrides, for example polyoxyethylene sorbitan mono-oleate. The said aqueous suspensions may also contain one or more preservatives, for example, ethyl or n-propyl p-hyroxy benzoate, one or more coloring agents, one or more flavoring agents and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oil suspensions may contain a thickening agent, for example beeswax, hard paraffin or cetyl alcohol. Sweetening agents, such as those set forth above, and flavoring agents may be added to

provide a palatable oral preparation. These compositions may be preserved by the addition of an antioxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of oil-in-water emulsions. The oily phase may be a vegetable oil, for example olive oil or arachis oils, or a mineral oil, for example liquid paraffin or mixtures of these. Suitable emulsifying agents may be naturally-occurring gums, for example gum acacia or gum tragacanth, naturally-occurring phosphatides, for example soya bean lecithin, and esters or partial esters derived from fatty acids and hexitol anhydrides, for example sorbitan mono-oleate, and condensation products of the said partial esters with ethylene oxide, for example polyoxyethylene sorbitan mono-oleate. The emulsions may also contain sweetening and flavoring agents.

Syrups and elixirs may be formulated with sweetening agents, for example glycerol, sorbitol or sucrose. Such formulations may also contain a

demulcent, a preservative and flavoring and coloring agents. The pharmaceutical compositions may be in the form of a sterile injectable preparation, for example as a sterile injectable aqueous or oleagenous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectibles.

The compounds of this invention may also be administered in the form of suppositories for rectal administration of the drug. These compositions can be prepared by mixing the drug with a suitable non-irritating excipient which is solid at ordinary temperatures by liquid at the rectal temperature and will thereofore melt in the rectum to release the drug. Such materials are cocoa butter and polyethylene glycols.

Dosage levels of the order of 1 to 500 mg per patient per day, in single or multiple doses, are

useful in the treatment of the above indicated conditions. Preferably, the dosage range will be from 5 to 100 mg per day.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

The compounds of this invention can also be administered in combination with other antihypertensives and/or diuretics. For example, the compounds of this invention can be given in combination with such compounds as amiloride, atenolol, bendroflumethiazide, chlorothalidone, chlorothiazide, clonidine, cryptenamine acetates and cryptenamine tannates, deserpidine, diazoxide, guanethidene sulfate, hydralazine hydrochloride, hydrochlorothiazaide, hydroflumethiazide, indacrinone, metolazone, metoprolol tartate, methylclothiazide, methyldopa, methyldopate hydrochloride, minoxidil, pargyline hydrochloride, polythiazide, prazosin, propranolol, <u>rauwolfia serpentina</u>, rescinnamine, reserpine, sodium nitroprusside, spironolactone, timolol,

trichlormethiazide, trimethophan camsylate, benzthiazide, quinethazone, ticrynafen, triamterene, acetazolamide, aminophylline, cyclothiazide, ethacrynic acid, furosemide, merethoxylline procaine, sodium ethacrynate, and the like, as well as admixtures and combinations thereof.

Typically, the individual daily dosages for these combinations can range from about one-fifth of the minimally recommended clinical dosages to the maximum recommended levels for the entities when they are given singly.

To illustrate these combinations, one of the antihypertensives of this invention effective in the 5 to 100 mg per day range can be effectively combined at levels at the 1 to 100 mg per day range with the following compounds at the indicated per day dose range: hydrochlorothiazide (10-100 mg); chlorothiazide (125-2000 mg); manipulated indacrinone enantiomer ratio (25-150 mg); ethacrynic acid (15-2000 mg); amiloride (5-20 mg); furosemide (5-80 mg); propranolol (20-480 mg); timolol (5-60 mg); and methyldopa (65-2000 mg); and the pivaloyloxyethyl ester of methyldopa (30-1000 mg). In addition, triple drug combinations of hydrochlorothiazide (10-100 mg) plus amiloride (5-20 mg) plus converting enzyme inhibitor of this invention (1-100 mg); hydrochlorothiazide (10-100 mg) plus timolol (5-60 mg) plus the converting enzyme inhibitor of this invention (1-100 mg); or manipulated indacrinone enantiomer ratio (25-150 mg) plus amiloride (5-20 mg) plus converting enzyme inhibitor of this invention

(1-100 mg) are effective combinations to control blood pressure in hypertensive patients. Naturally, these dose ranges can be adjusted on a unit basis as necessary to permit divided daily dosage and, as noted above, the dose will vary depending on the nature and severity of the disease, weight of patient, special diets and other factors.

Typically, these combinations can be formulated into pharmaceutical compositions as discussed below.

About 1 to 100 mg of a compound or mixture of compounds of Formula I or a physiologicaly acceptable salt is compounded with a physiologically acceptable vehicle, carrier, excipient, binder, preservative, stablizer, flavor, etc., in a unit dosage form as called for by accepted pharmaceutical practice. The amount of active substance in these compositons or preparations is such that a suitable dosage in the range indicated is obtained.

The compounds of Formula I can be prepared by one or more of the methods described further below.

As will be evident to those skilled in the art and as demonstrated in the Examples which follow, reactive groups not involved in the reactions, such as amino, carboxy, mercapto, etc., may be protected by methods standard in peptide chemistry prior to the coupling reactions and subsequently deprotected to obtain the desired products.

For most compounds of Formulae I and Ia, their preparation can be carried out in accordance with the Reaction Scheme illustrated below wherein R,

$R^1$, $R^3$, $R^8$, A, B, r and s are as defined above and R' and R" are independently hydrogen, aryl, heteroaryl, loweralkyl or loweralkyl substituted by halo, aryl, heteroaryl, alkoxy, alkylthio, dialkylamino, protected amino or protected alkylamino groups.

REACTION SCHEME

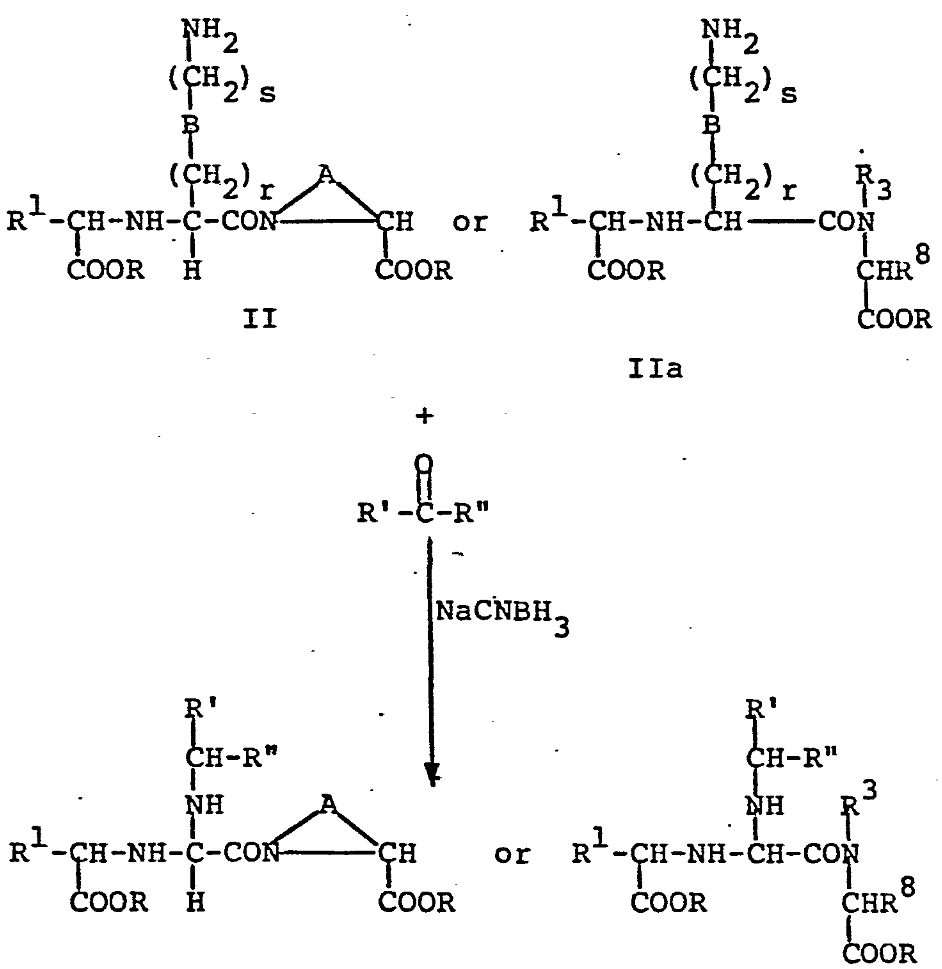


II or IIa

+

or

(III) (IIIa)

In the reaction illustrated above, a starting material of Formula II is reacted reductively with the appropriate aldehyde or ketone to form the desired product. The starting materials, which may be prepared in accordance with the procedures described in published European Pat. Appl. No. 0 012 401, are dissolved in a suitable solvent and the desired aldehyde or ketone is added, after which there is slowly added to the reaction mixture sodium cyanoborohydride. A preferred starting material is $N^2$-[1-(S)-carboxy-3-phenylpropyl]-L-lysyl-L-proline; which may be prepared in accordance with Example 57 of European Pat. App. No. 0 012 401 referred to above. Methylation using formic acid/formaldehyde is conveniently applied to N-benzyl intermediates which are then convertible to N-methyl products III or IIIa by hydrolysis of the benzyl group using a palladium catalyst in the presence of acetic acid.

An additional method for preparing products of this invention involves direct alkylation of compounds II or IIa with compounds having the formula: R'R"-CHX, wherein X is bromo, iodo, tosyloxy, mesyloxy, or similar leaving groups, and R' and R" are as defined above. These alkylations are carried out under basic conditions in solvents such as dimethylformamide or aqueous dimethylformamide. Mixtures of products and starting material are formed which can then be separated by liquid chromatography to yield products of this invention.

The following examples are provided to further illustrate the present invention, but not be a limitation thereof.

EXAMPLE 1

$N^2$-[1-(S)-Carboxy-3-phenylpropyl]-$N^6$-(phenylmethyl)-L-lysyl-L-proline

In 80 ml of methanol and 1 drop of water there was dissolved $N^2$-[1-(S)-carboxy-3-phenylpropyl]-L-lysyl-L-proline (600 mg, 1.48 mmol), after which there was added with stirring benzaldehyde (173 mg, 1.63 mmol) and 3A powdered molecular sieves (1.2 g). While the reaction mixture was stirred under a nitrogen atmosphere, there was added dropwise over 4 hrs sodium cyanoborohydride (102 mg, 1.63 mmol) dissolved in 4 ml of methanol. The reaction mixture was filtered through diatomaceous earth and concentrated under vacuum to 1.20 g. Product purification was carried out using 40 cc of Dowex 50 ($H^+$), eluting with 3% pyridine/water. Fractions 4-8 (100 ml) were collected and concentrated to 20 ml and freeze-dried (581 mg). Further purification was carried out on a 240 X 2.5 cm LH-20 chromatographic column eluting with 17 ml fractions of methanol. Fractions 28-32 were combined and concentrated to dryness. The residue was taken up in 25 ml of diethyl ether, stirred, allowed to stand overnight, and filtered to give a crystalline solid (490 mg). NMR ($CD_3OD$): $\delta$ 1.5-2.5 (br. mult. 12H, proline C-3 and C-4, $-CH\underline{CH_2}\underline{CH_2}\underline{CH_2}CH_2N-$, $-\underline{CH_2}CH_2Ph$), 4.12 (S, benzylmethylene), 7.07 (narrow mult., benzyl aromatic), 7.32 (narrow mult., phenethyl aromatic).

EXAMPLE 2

$N^2$-[1-(S)-Carboxy-3-phenylpropyl]-$N^6$-(methyl)-L-lysyl-L-proline

In 0.50 ml of formic acid there was dissolved $N^2$-[1-(S)-carboxy-3-phenylpropyl]-$N^6$-(phenylmethyl)-L-lysyl-L-proline (440 mg, 0.89 mmol) prepared in Example 1 above. The solution was heated to 100°C under a nitrogen atmosphere with stirring, after which 0.09 ml of 37% formaldehyde (1.1 mmol) was added dropwise over 1 min, followed by stirring of the reaction mixture for 15 min. The reaction mixture was then cooled to room temperature, 5 ml of acetic acid was added, and removal of the phenylmethyl group from the starting material was accomplished by well-known hydrogenation techniques using a palladium catalyst. The reaction mixture was then filtered and concentrated to about 0.5 ml, then triturated with 5 ml of diethylether. The reaction mixture was then put through a Dowex 50 chromatographic column, eluting with 25 ml fractions of 3% pyridine/water. Fractions 5-8 were concentrated and freeze-dried (360 mg), then further purified on a 230 X 2.5 cm LH-20 chromatographic column using a methanol system. The title compound was obtained from fractions 31-35 (318 mg). NMR ($CD_3OD$): $\delta$ 2.65 (d, $-NH\underline{CH_3}$), 7.20 (m, $C_6\underline{H_5}$), 7.40 (m, $-N\underline{H}CH_3$). The mass spectrum showed 562 m/e = ($M^+$ + 2TMS)-1, 349 m/e (loss of silyl proline).

EXAMPLE 3

Using known amino acids and known substituted 2-oxoalkanoic acids or their esters and employing the methods taught in Eur. Pat. Appln. No. 0 012 401, it is possible to synthesize starting materials for alkylation such as those illustrated in Table I hereinbelow.

Representative alkylating agents are shown in Table II hereinbelow. They are treated with an equivalent weight of compounds of Table I in the presence of excess base in a solvent such as aqueous DMF. The mixture of starting material and products is separated by HPLC to afford products of Formulas I and Ia listed in Table III hereinbelow.

Table II also lists aldehydes and ketones which may be reductively alkylated to the ω-amino group of compounds of Table I using sodium cyanoborohydride or by hydrogenation in the presence of catalysts such as Pd/C. Products of this type are also listed in Table III.

TABLE I

Starting Materials for Alkylation

a) 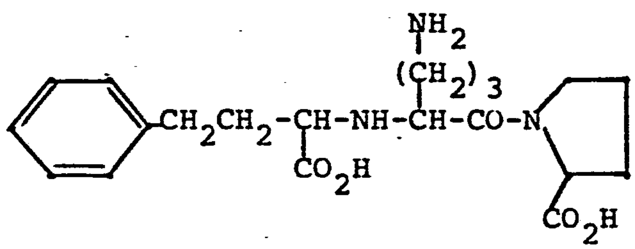

b) 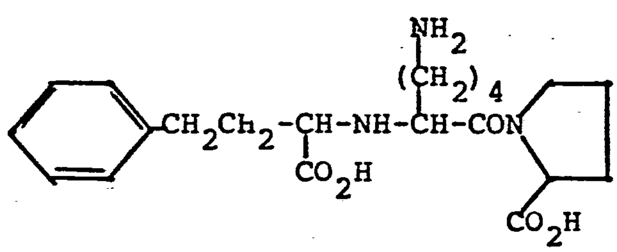

c) 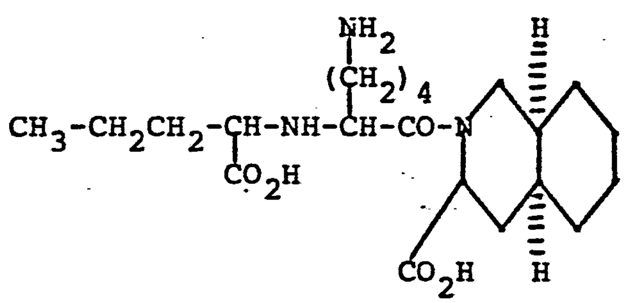

d) 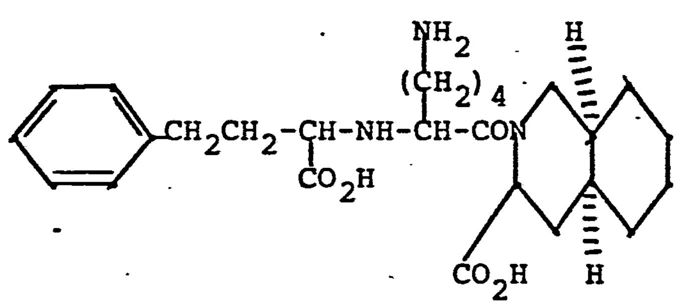

e) 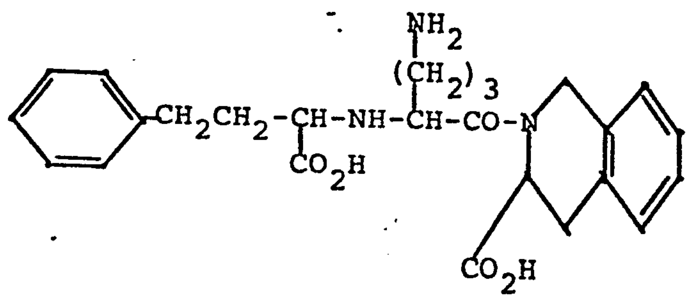

f) 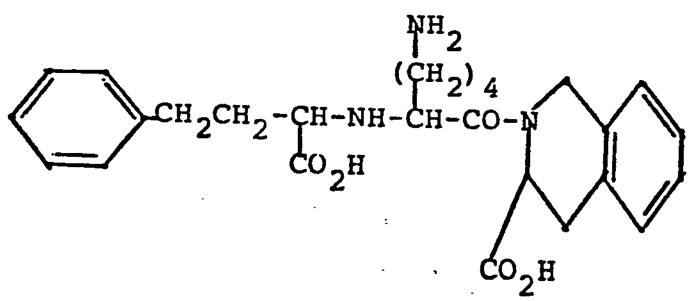

g) 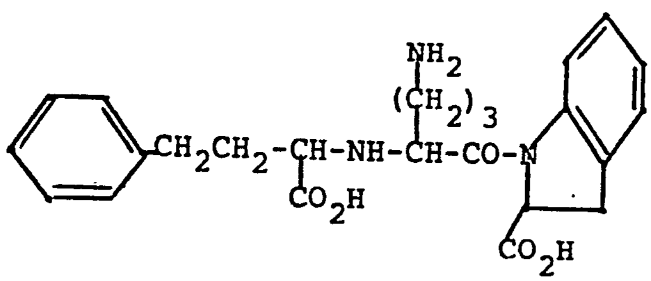

h) 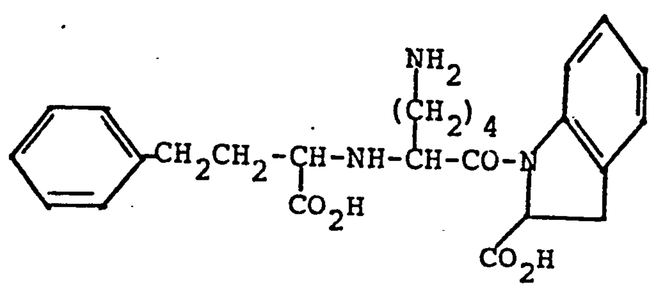

i) 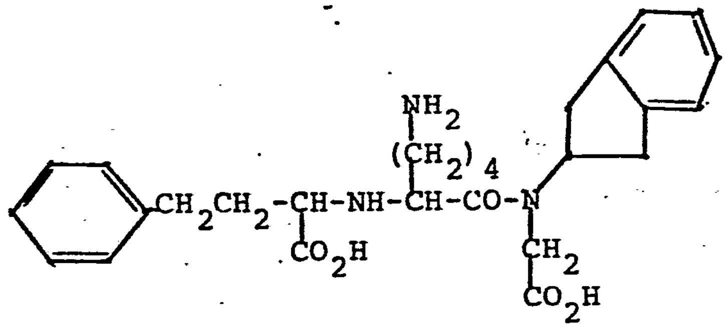

j) 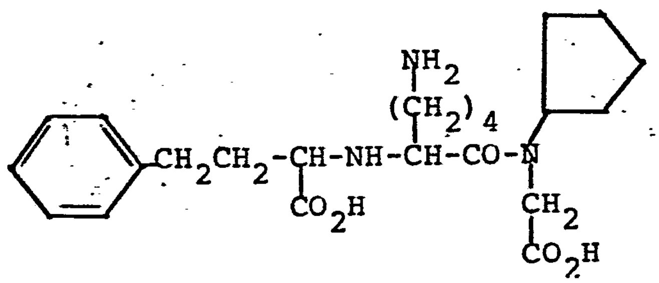

TABLE II

Reagents for N-Alkylation

A) $Cl-CH_2CH_2Br$

B) $CF_3CH_2-O-SO_2CH_3$

C) 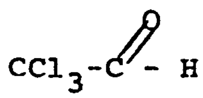

D) $HC=C-CH_2Br$

E) $CH_2=CH_2-CH_2Br$

F) $CH_3O-CH_2CH_2Cl$

G) $CH_3S-CH_2CH_2Cl$

H) 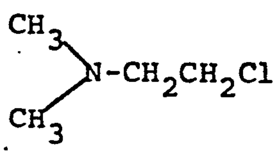

I) 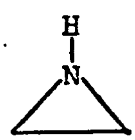

J) 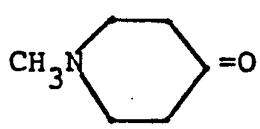

K) 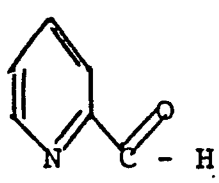

L) 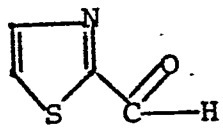

M) 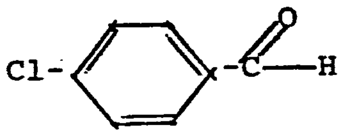

N) 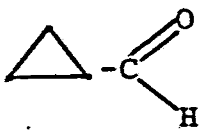

O) 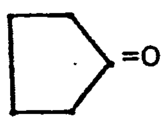

P) 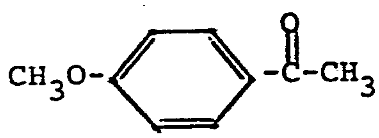

Q) 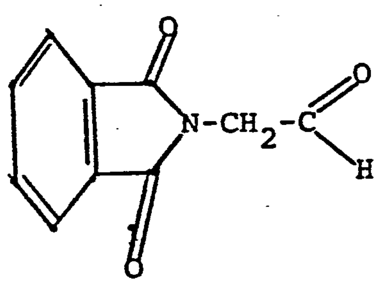

TABLE III

Products of Formulae I and Ia

1) 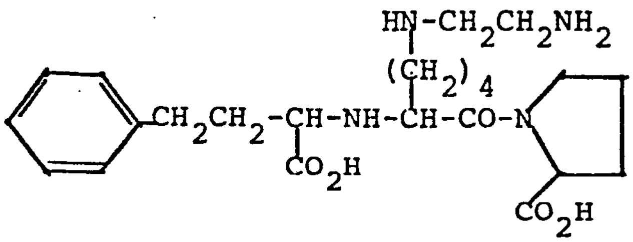

2) 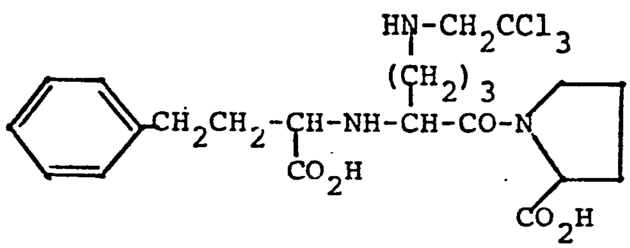

3) 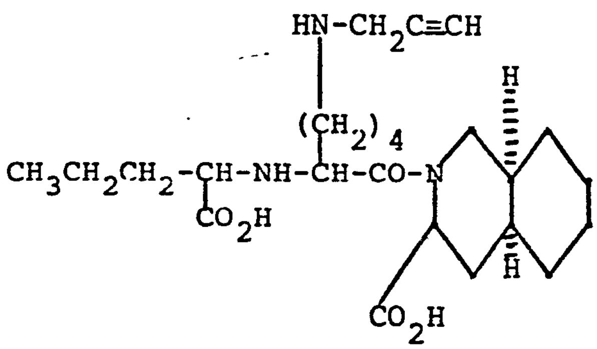

4) 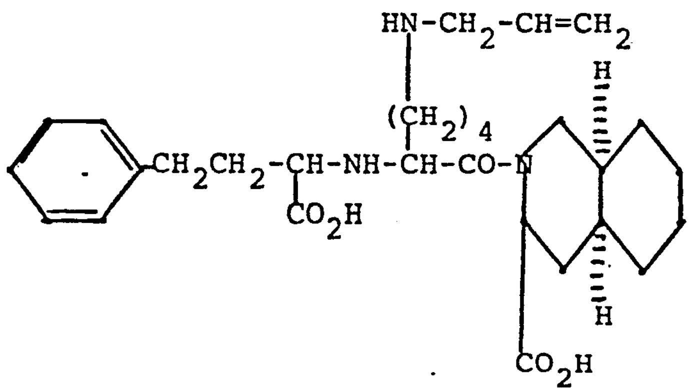

9) 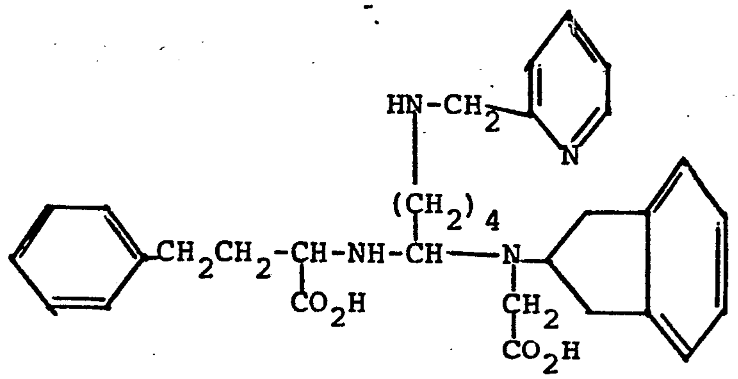

10) 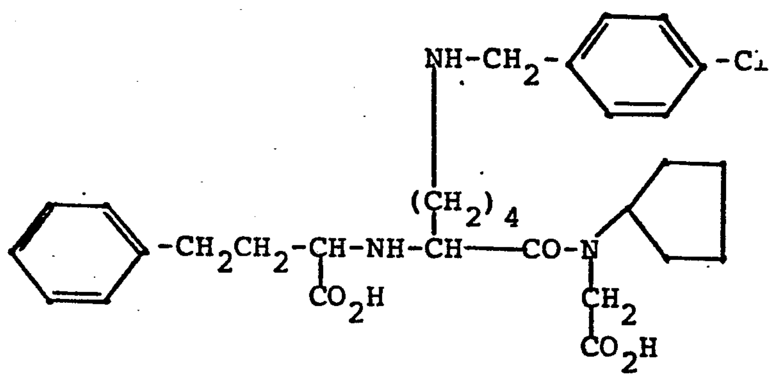

11) 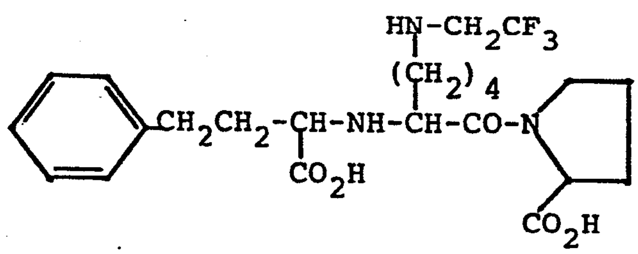

12) 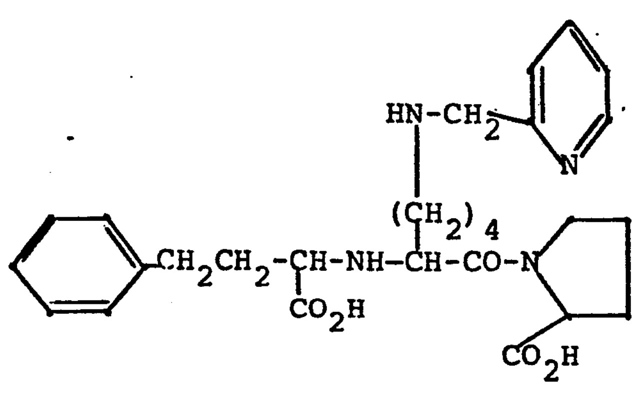

13) 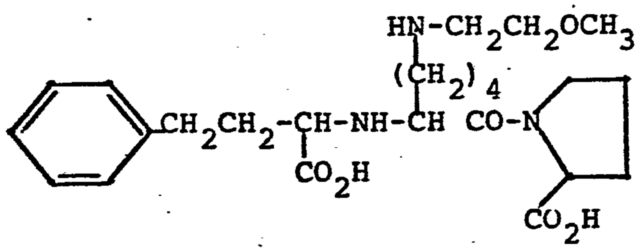

14) 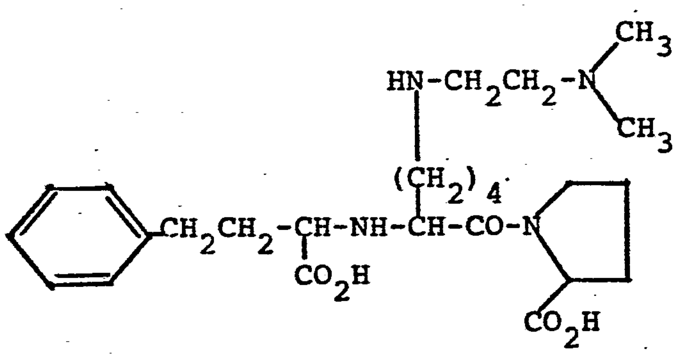

WHAT IS CLAIMED IS:

1. Compounds having the formulae:

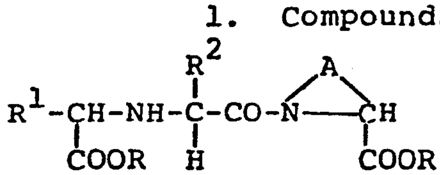 and 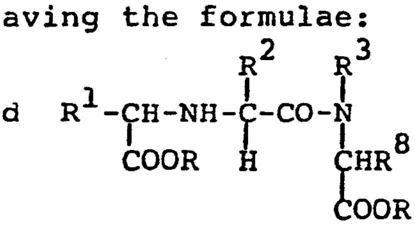

(I) (Ia)

wherein:

R is hydrogen; loweralkyl; aralkyl; aryl;

$R^1$ is hydrogen; $C_{1-12}$ alkyl; $C_{3-9}$ cycloalkyl; $C_{1-12}$ alkenyl; substituted loweralkyl where the substituents are halo, hydroxy loweralkoxy, aryloxy, amino, mono- or diloweralkylamino, acylamino, arylamino, guanidino, mercapto, loweralkylthio, arylthio, carboxy, carboxamido, or loweralkoxycarbonyl; aryl; substituted aryl where the substituents are loweralkyl, loweralkoxy, or halo; arloweralkyl; arloweralkenyl; heteroarloweralkyl; heteroarloweralkenyl; substituted arloweralkyl, substituted arloweralkenyl, substituted heteroarloweralkyl, or substituted heteroarloweralkenyl where the aryl and heteroaryl substituents are halo, loweralkyl, hydroxy, loweralkoxy, amino, aminoloweralkyl, acylamino, mono- or diloweralkylamino, carboxyl, haloloweralkyl, cyano, or sulfonamido, and where the loweralkyl portion of arloweralkyl may be substituted by amino, acylamino, or hydroxyl;

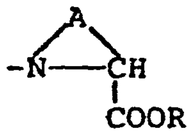 is 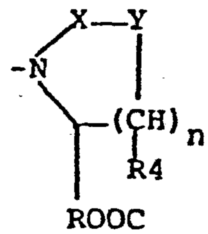, 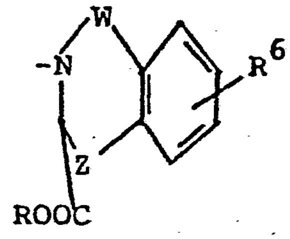

or 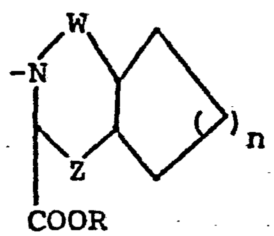

where

X and Y taken together are $-CH_2-CH_2-$;

$-CH(R^5)-S-$; $-C(=O)-CH_2-$; $-CH_2-C(=O)-$; $-C(=O)-O-$; $-C(=O)-S-$;

$-CH_2-CH(OR^4)-$; $-C(=O)-N(R^4)-$; or $-CH_2CR^4R^5-$

$R^4$ is hydrogen or loweralkyl;

$R^5$ is hydrogen; loweralkyl; aryl; or substituted aryl;

n is 1 to 3;

W is absent; $-CH_2-$; or $-C(=O)-$;

Z is $-(CH_2)_m-$, where m is 0 to 2, provided that m may not be 0 and W may not be absent at the same time; and

$R^6$ is hydrogen; loweralkyl; halo; or $OR^4$;

$R^3$ is $C_{3-8}$ cycloalkyl and benzofused derivatives thereof; aryl; heteroaryl; aralkyl; heteroaralkyl;

$R^8$ is hydrogen; loweralkyl;

$R^2$ is $-(CH_2)_r-B-(CH_2)_s-NHR^7$

where

r is 1-2;

s is 1-3;

B is absent; -O-; -S-; or $-NR^9-$;

where $R^9$ is hydrogen; loweralkyl; alkanoyl; or aroyl; and

$R^7$ is loweralkyl; loweralkenyl; loweralkynyl; substituted loweralkyl wherein the substituent is amino, alkyl and dialkylamino, halo, alkoxy or arylthio; aralkyl; substituted aralkyl wherein the substituent is halo, alkoxy, or hydroxy; heteroaralkyl; substituted heteroaralkyl wherein the substituent is amino, hydroxy, or loweralkyl;

and, a pharmaceutically acceptable salt thereof.

2. A compound of Claim 1 which is a member of the group:

$N^2$-[1-(S)-carboxy-3-phenylpropyl]-$N^6$-(methyl)-L-lysyl-L-proline;

$N^2$-[1-(S)-carboxy-3-phenylpropyl]-$N^6$-(2'-aminoethyl)-L-lysyl-L-proline;

$N^2$-[1-(S)-carboxy-3-phenylpropyl]-$N^6$-(2',2',2'-trifluoroethyl)-L-lysyl-L-proline;

$N^2$-[1-(S)-carboxy-3-phenylpropyl]-$N^6$-(2'-methoxyethyl)-L-lysyl-L-proline; and

$N^2$-[1-(S)-carboxy-3-phenylpropyl]-$N^6$-(2'-dimethylaminoethyl)-L-lysyl-L-proline.

3. A pharmaceutical composition useful in treating hypertension comprising a pharmaceutically acceptable carrier and an antihypertensively effective amount of a compound of the formulae:

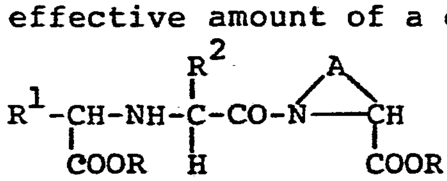

and

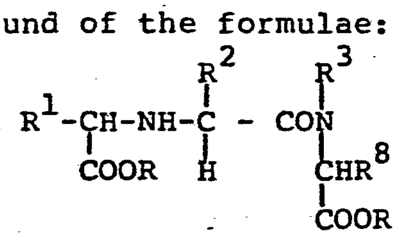

(I.) (Ia)

wherein:

R is hydrogen; loweralkyl; aralkyl; aryl;

$R^1$ is hydrogen; $C_{1-12}$ alkyl; $C_{3-9}$ cycloalkyl; $C_{1-12}$ alkenyl; substituted loweralkyl where the substituents are halo, hydroxy loweralkoxy, aryloxy, amino, mono- or diloweralkylamino, acylamino, arylamino, guanidino, mercapto, loweralkylthio, arylthio, carboxy, carboxamido, or loweralkoxycarbonyl; aryl; substituted aryl where the substituents are loweralkyl, loweralkoxy, or halo; arloweralkyl; arloweralkenyl; heteroarloweralkyl; heteroarloweralkenyl; substituted arloweralkyl, substituted arloweralkenyl,

substituted heteroarloweralkyl, or substituted heteroarloweralkenyl where the aryl and heteroaryl substituents are halo, loweralkyl, hydroxy, loweralkoxy, amino, aminoloweralkyl, acylamino, mono- or diloweralkylamino, carboxyl, haloloweralkyl, cyano, or sulfonamido, and where the loweralkyl portion of arloweralkyl may be substituted by amino, acylamino, or hydroxyl;

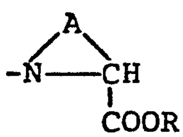 is 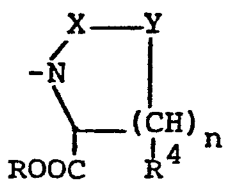, 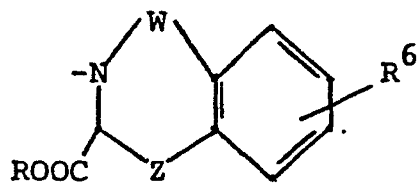

or 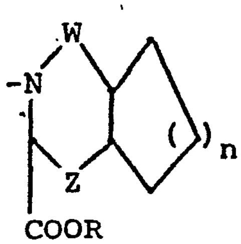

where

X and Y taken together are $-CH_2-CH_2-$;

$-\underset{R^5}{CH}-S-$; $-\underset{O}{\underset{\|}{C}}-CH_2-$; $-CH_2-\overset{O}{\overset{\|}{C}}-$; $-\overset{O}{\overset{\|}{C}}-O-$; $-\overset{O}{\overset{\|}{C}}-S-$;

$-CH_2-\overset{OR^4}{CH}-$; $-\overset{O}{\overset{\|}{C}}-\overset{R^4}{N}-$; or $-CH_2CR^4R^5-$

$R^4$ is hydrogen or loweralkyl;

$R^5$ is hydrogen; loweralkyl; aryl; or substituted aryl;

n is 1 to 3;

W is absent; $-CH_2-$; or $-\overset{O}{\overset{\|}{C}}-$;

Z is $-(CH_2)_m-$, where m is 0 to 2, provided that m may not be 0 and W may not be absent at the same time; and

$R^6$ is hydrogen; loweralkyl; halo; or $OR^4$;

$R^3$ is $C_{3-8}$ cycloalkyl and benzofused derivatives thereof; aryl; heteroaryl; aralkyl; heteroaralkyl;

$R^8$ is hydrogen; loweralkyl;

$R^2$ is $-(CH_2)_r-B-(CH_2)_s-NHR^7$

where

r is 1-2;

s is 1-3;

B is absent; -O-; -S-; or $-NR^9-$;

where $R^9$ is hydrogen; loweralkyl; alkanoyl; or aroyl; and

$R^7$ is loweralkyl; loweralkenyl; loweralkynyl; substituted loweralkyl wherein the substituent is amino, alkyl and dialkylamino, halo, alkoxy or arylthio; aralkyl; substituted aralkyl wherein the substituent is halo, alkoxy, or hydroxy; heteroaralkyl; substituted heteroaralkyl wherein the substituent is amino, hydroxy, or loweralkyl;

and, a pharmaceutically acceptable salt thereof.

4. A pharmaceutical composition useful in the treatment of hypertension which comprises a pharmaceutically acceptable carrier; an antihypertensively effective amount of a compound of Claim 1; and, an antihypertensive and/or diuretic compound selected from the group consisting of amiloride, atenolol, bendroflumethiazide, chlorothalidone, chlorothiazide, clonidine, cryptenamine acetates and cryptenamine tannates, deserpidine, diazoxide, guanethidene sulfate, hydralazine hydrochloride, hydrochlorothiazaide, hydroflumethiazide, indacrinone, metolazone, metoprolol tartate, methylclothiazide, methyldopa, methyldopate hydrochloride, minoxidil, pargyline hydrochloride, polythiazide, prazosin, propranolol, rauwolfia serpentina, rescinnamine, reserpine, sodium nitroprusside, spironolactone, timolol, trichlormethiazide, trimethophan camsylate, benzthiazide, guinethazone, ticrynafen, triamterene, acetazolamide, aminophylline, cyclothiazide, ethacrynic acid, furosemide, merethoxylline procaine and sodium ethacrynate.

5. A process for preparing compounds having the formulae:

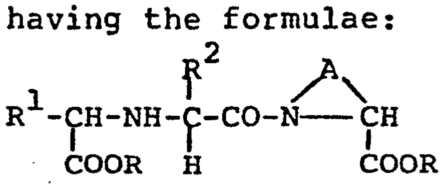

and

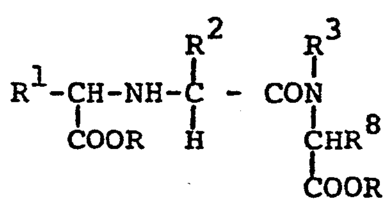

(I.) (Ia)

wherein:

R is hydrogen; loweralkyl; aralkyl; aryl;

$R^1$ is hydrogen; $C_{1-12}$ alkyl; $C_{3-9}$ cycloalkyl; $C_{1-12}$ alkenyl; substituted loweralkyl where the substituents are halo, hydroxy loweralkoxy, aryloxy, amino, mono- or

diloweralkylamino, acylamino, arylamino, guanidino, mercapto, loweralkylthio, arylthio, carboxy, carboxamido, or loweralkoxycarbonyl; aryl; substituted aryl where the substituents are loweralkyl, loweralkoxy, or halo; arloweralkyl; arloweralkenyl; heteroarloweralkyl; heteroarloweralkenyl; substituted arloweralkyl, substituted arloweralkenyl, substituted heteroarloweralkyl, or substituted heteroarloweralkenyl where the aryl and heteroaryl substituents are halo, loweralkyl, hydroxy, loweralkoxy, amino, aminoloweralkyl, acylamino, mono- or diloweralkylamino, carboxyl, haloloweralkyl, cyano, or sulfonamido, and where the loweralkyl portion of arloweralkyl may be substituted by amino, acylamino, or hydroxyl;

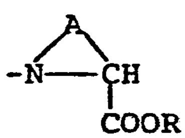 is 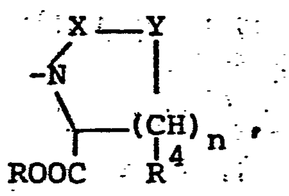, 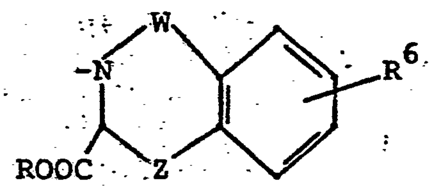

or 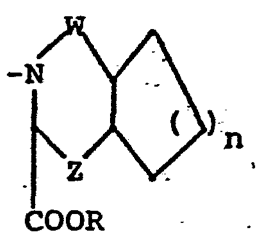

where

X and Y taken together are $-CH_2-CH_2-$;

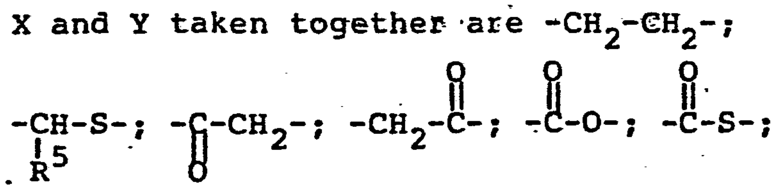

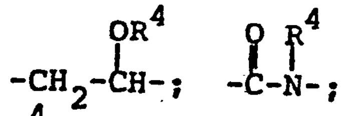 or $-CH_2CR^4R^5-$

$R^4$ is hydrogen or loweralkyl;

$R^5$ is hydrogen; loweralkyl; aryl; or substituted aryl;

n is 1 to 3;

W is absent; $-CH_2-$; or 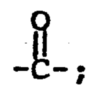

Z is $-(CH_2)_m-$, where m is 0 to 2, provided that m may not be 0 and W may not be absent at the same time; and

$R^6$ is hydrogen; loweralkyl; halo; or $OR^4$;

$R^3$ is $C_{3-8}$ cycloalkyl and benzofused derivatives thereof; aryl; heteroaryl; aralkyl; heteroaralkyl;

$R^8$ is hydrogen; loweralkyl;

$R^2$ is $-(CH_2)_r-B-(CH_2)_s-NHR^7$

where

r is 1-2;

s is 1-3;

B is absent; -O-; -S-; or $-NR^9-$; where $R^9$ is hydrogen; loweralkyl; alkanoyl; or aroyl; and

$R^7$ is loweralkyl; loweralkenyl; loweralkynyl; substituted loweralkyl wherein the substituent is amino, alkyl and dialkylamino, halo, alkoxy or arylthuo; aralkyl; substituted aralkyl wherein the substituent is halo, alkoxy, or hydroxy; heteroaralkyl; substituted heteroaralkyl wherein the substituent is amino, hydroxy, or loweralkyl;

which process comprises:

reacting a compound having the formulae:

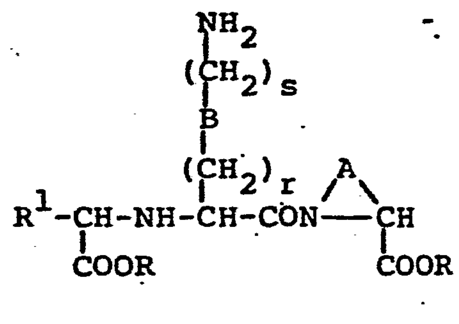 or 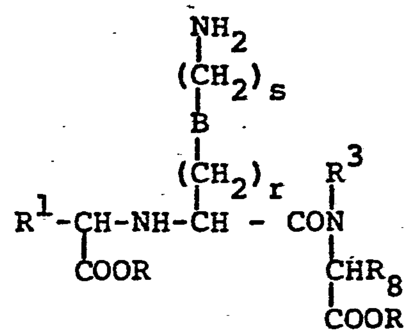

(II) (IIa)

wherein R, $R^1$, $R^3$, $R^8$, A, B, r and s are as defined above, with a compound of the formula:

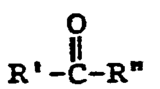 wherein R' and R" are independently hydrogen, aryl, heteroaryl, loweralkyl, or loweralkyl substituted by halo, aryl, heteroaryl, alkoxy, alkylthio, dialkylamino, protected amino or protected alkylamino groups, in the presence of cyanoborohydride followed by the removal of protecting groups, if any, to obtain said formulae I or Ia compounds and, if desired, forming a salt thereof by conventional means.

6. A process for preparing compounds having the formulae:

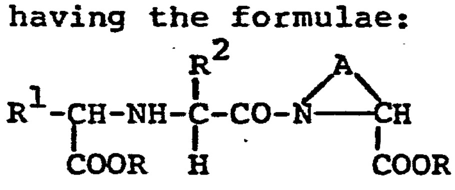 and 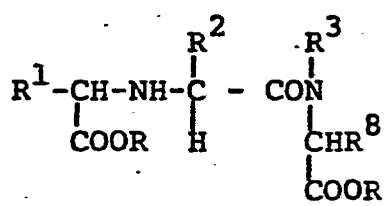

(I.) (Ia)

wherein:

R is hydrogen; loweralkyl; aralkyl; aryl;

$R^1$ is hydrogen; $C_{1-12}$ alkyl; $C_{3-9}$ cycloalkyl; $C_{1-12}$ alkenyl; substituted loweralkyl where the substituents are halo, hydroxy loweralkoxy, aryloxy, amino, mono- or diloweralkylamino, acylamino, arylamino, guanidino, mercapto, loweralkylthio, arylthio, carboxy, carboxamido, or loweralkoxycarbonyl; aryl; substituted aryl where the substituents are loweralkyl, loweralkoxy, or halo; arloweralkyl; arloweralkenyl; heteroarloweralkyl; heteroarloweralkenyl; substituted arloweralkyl, substituted arloweralkenyl, substituted heteroarloweralkyl, or substituted heteroarloweralkenyl where the aryl and heteroaryl substituents are halo, loweralkyl, hydroxy, loweralkoxy, amino, aminoloweralkyl, acylamino, mono- or diloweralkylamino, carboxyl, haloloweralkyl, cyano, or sulfonamido, and where the loweralkyl portion of arloweralkyl may be substituted by amino, acylamino, or hydroxyl;

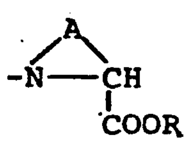 is 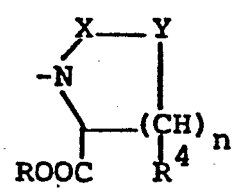 , 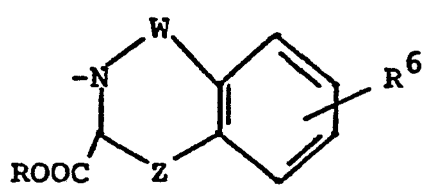

or 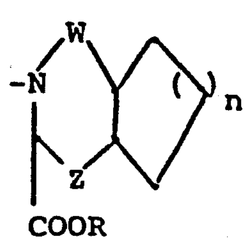

where

X and Y taken together are $-CH_2-CH_2-$;

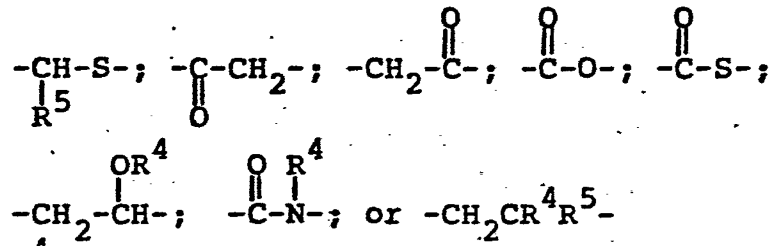

or $-CH_2CR^4R^5-$

$R^4$ is hydrogen or loweralkyl;

$R^5$ is hydrogen; loweralkyl; aryl; or substituted aryl;

n is 1 to 3;

W is absent; $-CH_2-$; or $-\overset{O}{\overset{\|}{C}}-$;

Z is $-(CH_2)_m-$, where m is 0 to 2, provided that m may not be 0 and W may not be absent at the same time; and

$R^6$ is hydrogen; loweralkyl; halo; or $OR^4$;

$R^3$ is $C_{3-8}$ cycloalkyl and benzofused derivatives thereof; aryl; heteroaryl; aralkyl; heteroaralkyl;

$R^8$ is hydrogen; loweralkyl;

$R^2$ is $-(CH_2)_r-B-(CH_2)_s-NHR^7$

where

r is 1-2;

s is 1-3;

B is absent; -O-; -S-; or $-NR^9-$;

where $R^9$ is hydrogen; loweralkyl; alkanoyl; or aroyl; and

$R^7$ is loweralkyl; loweralkenyl; loweralkynyl; substituted loweralkyl wherein the substituent is amino, alkyl and dialkylamino, halo, alkoxy or

arylthio; aralkyl; substituted aralkyl wherein the substituent is halo, alkoxy, or hydroxy; heteroaralkyl; substituted heteroaralkyl wherein the substituent is amino, hydroxy, or loweralkyl;

which process comprises:

reacting a compound having the formulae:

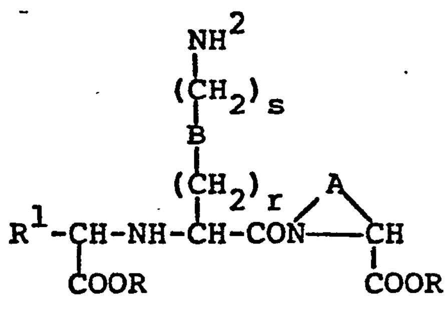 or 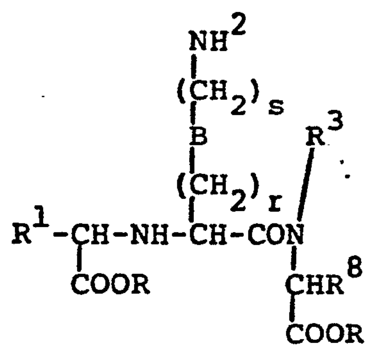

(II) (IIa)

wherein R, R1, R3, R8, A, B, r and s are as defined above, with a compound of the formulae: R'R"-CH-X wherein X is a leaving group selected from bromo, iodo, mesyloxy, or toluene sulfonyloxy groups, and R' and R" are independently hydrogen, aryl, heteroaryl, loweralkyl, or loweralkyl substituted by halo, aryl, heteroaryl, alkoxy, arylthio, dialkylamino, protected amino, or protected alkylamino groups, in the presence of a base in a solvent such as dimethyl formamide, to obtain a mixture containing said formulae I, Ia, II and IIa compounds; separating said formulae I and Ia compounds from said mixture by chromatography; removing protecting groups therefrom, if any; and, if desired, forming a salt thereof by conventional means.

European Patent Office

# EUROPEAN SEARCH REPORT

0081094

Application number

EP 82 11 0398

**DOCUMENTS CONSIDERED TO BE RELEVANT**

| Category | Citation of document with indication, where appropriate, of relevant passages | Relevant to claim | CLASSIFICATION OF THE APPLICATION (Int Cl 3) |
|---|---|---|---|
| D,A | EP-A-0 012 401 (MERCK & CO.) * Examples * | 1-5 | C 07 C 103/52<br>A 61 K 37/02 |
| A | EP-A-0 018 549 (TANABE SEIYAKU) * Examples * | 1-5 | |
| P,A | EP-A-0 049 506 (MERCK & CO.) | | |
| P,A | EP-A-0 049 605 (WARNER-LAMBERT CO.) | | |
| P,A | EP-A-0 050 800 (SCHERING CORP.) * Example 44; page 77 line 17; page 86, lines 23- 27; page 96, line 36 - page 97, line 6 * | 1-5 | TECHNICAL FIELDS SEARCHED (Int. Cl. 3) |
| P,A | EP-A-0 058 918 (MERCK & CO.) * Examples 39, 44 * | 1-5 | C 07 C 103/52<br>C 07 D 217/26 |
| P,A | EP-A-0 061 186 (MERCK & CO.) * Examples * | 1-5 | |

The present search report has been drawn up for all claims

| Place of search | Date of completion of the search | Examiner |
|---|---|---|
| BERLIN | 02-02-1983 | BREW C.H. |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document